(19) Europäisches Patentamt
European Patent Office
Office européen des brevets

(11) EP 0 623 103 B1

(12) EUROPÄISCHE PATENTSCHRIFT

(45) Veröffentlichungstag und Bekanntmachung des Hinweises auf die Patenterteilung:
**03.09.1997 Patentblatt 1997/36**

(21) Anmeldenummer: **93902218.2**

(22) Anmeldetag: **15.01.1993**

(51) Int Cl.[6]: **C07C 45/30**, C07C 49/80, C07C 49/84, C07C 49/327, C07D 213/61, C07D 213/64, C07D 333/28, C07D 213/50

(86) Internationale Anmeldenummer:
**PCT/EP93/00094**

(87) Internationale Veröffentlichungsnummer:
**WO 93/14054 (22.07.1993 Gazette 1993/18)**

# (54) VERFAHREN ZUR HERSTELLUNG VON TRIFLUORMETHYLKETONEN

PROCESS FOR PRODUCING TRIFLUOROMETHYL KETONES

PROCEDE DE PREPARATION DE TRIFLUOROMETHYLCETONES

(84) Benannte Vertragsstaaten:
**CH DE FR GB LI NL**

(30) Priorität: **21.01.1992 DE 4201435**

(43) Veröffentlichungstag der Anmeldung:
**09.11.1994 Patentblatt 1994/45**

(73) Patentinhaber: **Hoechst Schering AgrEvo GmbH
13509 Berlin (DE)**

(72) Erfinder:
- **DÖLLER, Uwe
D-6054 Rodgau (DE)**
- **SCHARBERT, Bernd
D-6230 Frankfurt am Main (DE)**
- **WEISSE, Laurent
D-6370 Niederursel (DE)**

(56) Entgegenhaltungen:
**US-A- 3 996 259**

- **TETRAHEDRON LETTERS Bd. 28, Nr. 37, 1987, OXFORD GB Seiten 4259 - 4262 R.J. LINDERMAN ET AL. 'An Efficient Procedure for the Oxidation of Fluorinated Carbinols'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 623 103 B1

## Beschreibung

Gegenstand der vorliegenden Erfindung ist ein Verfahren zur Herstellung von Trifluormethylketonen der Formel I

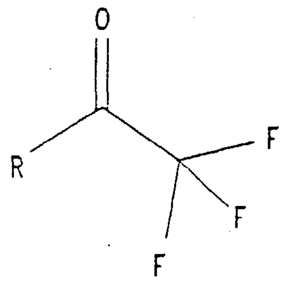

( I )

worin R ein unsubstituierter oder substituiert aliphatischer oder aromatischer Kohlenwasserstoffrest oder ein unsubstituierter oder substituierter aliphatisch- oder aromatisch-heterocyclischer Rest ist,
dadurch gekennzeichnet, daß man einen Alkohol der Formel II,

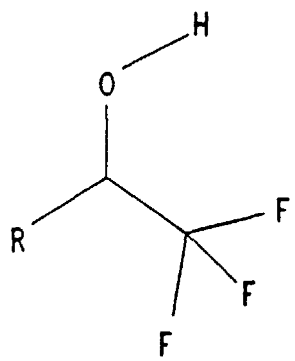

( I I )

worin R die oben genannte Bedeutung besitzt, in einem aprotischen Lösemittel, vorzugsweise Methylenchlorid, Chloroform, Ethylacetat, Toluol, Xylol oder Chlorbenzol, in Gegenwart von 0,1 - 20 Mol.-%, vorzugsweise von 1 - 10 Mol.-%, bezogen auf den eingesetzten Alkohol der Struktur II, eines
Phasentransferkatalysators mit einer 1 - 20 gew.-%igen wäßrigen Lösung einer Verbindung der Struktur IV oder einer 1 - 20 gew.-%igen wäßrigen Lösung einer in situ erzeugten Verbindung der Struktur IV

$$YO_nH \qquad (IV)$$

worin Y = Cl, Br oder J und n = eine ganze Zahl von 1 bis 4 bedeuten, oder von deren Salzen bei Temperaturen von 0°C bis zum Siedepunkt der Mischung, umsetzt.

Die Verbindungen der Formel I sind wichtige Vor- und Zwischenprodukte, z.B. bei der Synthese von Pharmaka, Pflanzenschutzmitteln (EP-A-0 386 715), Kunststoffen (US-Patentschrift 3,342,778), oberflächenaktiven Substanzen (DD 2397 88 A1), Materialien für nichtlineare Optik (WO 91/08198), Flüssigkristallen und Farbstoffen.

Bekannt ist, daß Trifluormethylalkohole gegenüber einer Vielzahl von Oxidationsmitteln inert sind (Tetrahedron (1991), 47 (20/21), 3207).

Umsetzungen von Alkoholen der Struktur II mit Dimethylsulfoxid in Gegenwart von Oxalylchlorid (AU-B-52 881/86) bei -55°C zu den Ketonen I erfordern mehr als 3 Mol des Oxidationsmittels pro Mol der eingesetzten Alkohole II. Die Reaktionsführung muß für den technischen Maßstab als nachteilig gelten. Die Verwendung von großen Mengen Oxalylchlorid ist aus ökonomischen Gründen nicht akzeptabel. Das bei der Umsetzung entstehende Dimethylsulfid ist aus ökologischer Sicht nachteilig zu bewerten.

Die Herstellung von Trifluormethylketonen der Struktur I durch Oxidation der Alkohole der Formel II mittels Dess-Martin-Reagenz (J. Org. Chem. (1989), 54, 661) erfordert den Einsatz aufwendiger, kommerziell nicht verfügbarer und sicherheitstechnisch bedenklicher, hypervalenter Jodverbindungen. Insbesondere die technische Verwendung von

Dess-Martin-Reagenz ist aus ökonomischer und sicherheitstechnischer Sicht nachteilig zu bewerten.

Der Einsatz von alkalischer Permanganatlösung (Tetrahedron Lett. (1986), 27(2), 135) zur Oxidation von Verbindungen der Struktur II beschränkt sich auf wasserlößliche Trifluormethylalkohole und liefert wenig reproduzierbare Ergebnisse (J. Org. Chem. 1989, 54, 661).

Die Verwendung von Chromsäuren zur Synthese von Verbindungen des Typs I aus den Alkoholen II (J. Org. Chem. (1968), 33(3), 1016) erfordert drastische Reaktionsbedingungen und mehrtägige Reaktionszeiten und läßt sich daher vorteilhaft nur auf innerte, perhalogenierte Alkohole anwenden. Die anfallenden Abfallchromsalze sind aus ökonomischer und ökologischer Sicht nachteilig zu bewerten.

Alle diese geschilderten Nachteile werden durch das erfindungsgemäße Verfahren, welches sehr einfach zu handhaben und auch für einen kontinuierlichen Betrieb geeignet ist, vermieden. Die Produkte I können unter den erfindungsgemäßen Bedingungen mittels leicht zugänglichen, ökonomisch vorteilhaften Oxidationsmittel der Struktur IV in hohen Ausbeuten z.B. bis zu 90 % d. Th. und in hoher Reinheit erhalten werden. Der Rest R kann hierbei sehr weit variiert werden.

R in Formel I enthält als Kohlenwasserstoffrest bevorzugt 1 bis 30, besonders 1 bis 20 und insbesondere 1 bis 12 C-Atome, R als heterocyclischer Rest enthält bevorzugt 1 bis 4 Heteroatome im Ring. Der Ring enthält bevorzugt 4 bis 8, besonders 5 oder 6 Ringglieder. Die Heteroatome sind bevorzugt O, S oder N und es können gleiche oder verschiedene Heteroatome im Ring enthalten sein.

R ist als aliphatischer Kohlenwasserstoffrest bevorzugt unsubstituiertes oder substituiertes Alkyl, Alkenyl, Alkinyl, Cycloalkyl oder Cycloalkenyl, als unsubstituierter oder substituierter aromatischer Kohlenwasserstoffrest Aryl, Aralkenyl, Aralkinyl oder Aralkyl, als unsubstituierter oder substituierter aliphatisch-heterocyclischer Rest ein 3- bis 7-gliedriger, 1 bis 3 Heteroatome enthaltender heterocyclischer Ring und als aromatisch-heterocyclischer Rest ein 5- oder 6-gliedriger, 1 bis 4 Heteroatome enthaltender heterocyclischer Ring.

Beispiele für Alkyl sind Methyl, Ethyl, n-Propyl, iso-Propyl, n-, iso- und tertiär-Butyl, Pentyl, Hexyl, Octyl, 2-Ethylhexyl, Nonyl, Decyl, Undecyl, Dodecyl, Tetradecyl, Octadecyl und Eicosyl.

Beispiele für Alkenyl sind Vinyl, Allyl, 1- oder 2-Methylvinyl, Styryl, Butenyl, Pentenyl, Hexenyl, Octenyl, Decenyl und Dodecenyl.

Beispiele für Alkinyl sind Ethinyl, Propargyl, Methylethinyl, Phenylethinyl, Butinyl, Pentinyl, Hexinyl, Octinyl, Decinyl und Dodecinyl.

Das Cycloalkyl und Cycloalkenyl kann 3 bis 12, vorzugsweise 3 bis 8 und insbesondere 3 bis 6, Ringkohlenstoffatome enthalten. Beispiele sind Cyclopropyl, Cyclobutyl, Cyclopentyl Cyclohexyl, Cycloheptyl, Cyclooctyl, Cyclononyl, Cyclodecyl, Cyclododecyl, Cyclopropenyl, Cyclobutenyl, Cyclopentadienyl, Cyclohexenyl, Cyclohexadienyl, Cyclooctenyl, Cyclooctadienyl, Bicyclo-2,2,2-octenyl, Bicyclo-2,2,1-heptenyl.

Der aromatische Kohlenwasserstoffrest kann ein- oder mehrkernig, vorzugsweise mono- oder bicyclisch, und kondensiert sein. Bevorzugt sind Aryl, Aralkenyl, Aralkinyl oder Aralkyl mit insbesondere 6 bis 18 bzw. 7 bis 18, bzw. 8 bis 18 C-Atomen. Das Aryl ist bevorzugt Phenyl oder Naphthyl. Beispiele sind Phenyl, Naphthyl, Benzyl, Phenylethyl, 2-Phenylpropyl, Naphthylmethyl, Dihydronaphthalin, Indan, Inden, Fluoren, Phenanthren.

R als aliphatisch-heterocyclischer Rest hat vorzugsweise 4 bis 6 Ringglieder und enthält, je nach Ringgröße, 1 bis 3, bevorzugt 1 oder 2, Heteroatome wie O, S oder N. Beispiele für Heteroringe, von denen sich der Rest R ableitet, sind: Oxetan, Oxolan, Oxolen, Oxan, Dioxan, Aziridin, Azetidin, Azetin, Pyrrolidin, Pyrrolin, Tetrahydrothiophen, 2,3-Dihydroindol, Dihydrocumaron, Dihydrothionaphthen, Pyrazolidin, Imidazolidin, Oxazolidin, Thiazolidin, Triazolidin, Oxadiazolidin, Morpholin, Piperidin, Tetrahydrochinolin.

R als aromatisch-heterocyclischer Rest enthält bevorzugt 1 bis 4, besonders 1 bis 2, Heteroatome wie O, S und N und stellt bevorzugt einen 5- oder 6-gliedrigen Ring dar. Ferner kommen auch kondensierte Ringsysteme in Frage. Beispiele für Heteroaromaten, von denen sich R ableiten kann, sind: Pyrrol, Furan, Thiophen, Pyridin, Pyran, Pyrazol, Imidazol, Benzimidazol, Triazin, Oxazol, Thiazol, Pyridazin, Pyrimidin, Pyrazin, Chinolin, Isochinolin, Chromen, Purin, Xanthen und Indol.

Die Reste R sind unsubstituiert oder ein- bis mehrfach substituiert, insbesondere 1-bis 5-fach. Die Anzahl der Substituenten ist u.a. abhängig von der Größe der Aromaten. Im Falle der mehrfachen Substitution sind die Substituenten gleich oder verschieden. Die Substituenten können ihrerseits substituiert sein.

Als Substituenten für den Rest R eignen sich solche Reste, die unter den Reaktionsbedingungen nich angegriffen werden.

Geeignete Substituenten für R sind zum Beispiel:

$C_1$-$C_{18}$-Alkyl, $C_2$-$C_{18}$-Alkenyl, $C_2$-$C_{18}$-Alkinyl, $C_6$-$C_{18}$-Aryl, $C_7$-$C_{18}$-Aralkyl, $C_7$-$C_{18}$-Alkaryl, $C_8$-$C_{18}$Alkarylalkyl, $C_1$-$C_{18}$-Alkoxy, $C_1$-$C_{18}$-Alkylthio, $C_6$-$C_{18}$-Aryloxy, $C_7$-$C_{18}$-Aralkoxy, $C_7$-$C_{18}$-Aralkylthio, $C_7$-$C_{18}$-Alkaryloxy, $C_8$-$C_{18}$-Alkaralkoxy, $C_7$-$C_{18}$-Aryloxyalkyl, $C_7$-$C_{18}$-Alkoxyaryl, Hetaryl, Hetarylalkyl, Alkylhetaryl, Alkylhetarylalkyl, Hetaryloxy, Hetarylalkyloxy, Hetarylalkylthio, Alkylhetaryloxy, Alkoxyhetaryl, Alkylhetarylalkyloxy, Hetaryloxyalkyl, wobei die heterocyclischen Arylreste jeweils bis zu 8 Kohlenstoffatome und bis zu 4 Heteroatome ausgewählt aus der Gruppe bestehend aus N, S und O, aufweisen und die gegebenenfalls anwesenden Alkylgruppen insgesamt bis zu 12 Kohlenstoffatome besitzen,

$C_3$-$C_{12}$-Cycloalkyl, $C_3$-$C_{12}$-Cycloalkoxy, $C_3$-$C_{12}$-Cycloalkenyl, C3-C12-Cycloalkenyloxy, $C_1$-$C_{18}$-Haloalkyl, insbesondere Fluoralkyl, -CN, OH, $NO_2$, F, Cl, Br, J, -NCO, $COOR^1$, $COR^1$, $-OCOR^1$, $-CONR^1R^2$, $-NR^1COR^2$, $-NR^1R^2$, $-SO_2NR^1R^2$, $-Si(R^1)_3$, $-SO_2R^1$, $-SO_3R^1$, $-PO_2R^1$, $-PO_3R^1R^2$. $R^1$ steht in den vorstehend aufgeführten Substituenten für H, $C_1$-$C_{18}$-Alkyl $C_1$-$C_{18}$-Haloalkyl, insbesondere für $C_1$-$C_{18}$-Fluoralkyl, $C_3$-$C_6$-Cycloalkyl, Phenyl, Benzyl oder Pyridyl; $R^2$ hat unabhängig die gleiche Bedeutung wie $R^1$.

Die aliphatischen Reste R können durch O, S, $NR^1$, worin $R^1$ die oben angegebene Bedeutung besitzt, -CO- oder -C(O)O- unterbrochen sein.

Als Substituent für R ist Aryl bevorzugt Phenyl oder Naphthyl, Hetaryl ist bevorzugt Pyridyl, Pyrimidyl oder Pyridazinyl, Thiazolyl oder Imidazolyl. Bevorzugte Substituenten der genannten aromatischen Reste sind $C_1$-$C_{12}$-Alkyl, Phenyl, Benzyl, $C_7$-$C_{14}$-Alkylphenyl, $C_8$-$C_{14}$-Alkylbenzyl, $C_1$-$C_{12}$-Alkoxy, Phenoxy, Benzyloxy, $C_7$-$C_{12}$-Alkylphenoxy, $C_8$-$C_{14}$-Alkylbenzyloxy, $C_7$-$C_{12}$-Phenoxyalkyl, $C_7$-$C_{14}$-Alkoxyphenyl, Cyclopentyl, Cyclohexyl, Cyclopropyl, $C_1$-$C_{12}$-Haloalkyl, insbesondere $C_1$-$C_{12}$-Fluoralkyl, $C_1$-$C_{12}$-Haloalkoxy, insbesondere $C_1$-$C_{12}$-Fluoralkoxy, CN, OH, F, Cl, Br, J, wobei die aliphatischen Reste wie zuvor definiert unterbrochen sein können.

Einige Beispiele für Substituenten am Rest R sind: Methyl, Ethyl, Propyl, Butyl, Vinyl, Allyl, Ethinyl, Propargyl, Phenyl, Benzyl, Methylbenzyl, Methylphenyl, Methoxy, Ethoxy, Propoxy, Butoxy, Methylthio, Phenoxy, Benzyloxy, Methylphenoxy, Methylbenzyloxy, Phenoxymethyl, Methoxyphenyl, Cyclopentyl, Cyclohexyl, Cyclohexenyl, Cyclohexyloxy, Methoxycarbonyl, Methylamino, Dimethylamino, Methylaminocarbonyl, Alkoxyalkyl wie Methoxymethyl oder Methoxyethyl, Alkylaminoalkyl wie Methylaminomethyl oder Dimethylaminoethyl, Phenoxyphenyl, Methoxycarbonylmethyl, Ethoxycarbonylethyl, Haloalkyl wie Chlormethyl, Dichlormethyl, Trichlormethyl, Fluormethyl, Difluormethyl, Trichlorethyl, Trifluormethyl, Hexafluorpropyl, Trifluorethyl, Haloalkoxy wie Monofluormethoxy, Difluormethoxy, Trifluormethoxy, Trifluorethoxy, Trichlorethoxy oder Hexafluorpropoxy, Haloaryl wie Fluorphenyl, Chlorphenyl, Haloaryloxy wie Fluorphenoxy, Chlorphenoxy, Haloalkoxyaryl wie Trifluormethoxyphenyl, Trifluorethoxyphenyl, sowie Trifluormethylphenyl, Trifluormethylphenoxy, Trifluorethoxypyridyl.

In einer bevorzugten Untergruppe ist R $C_1$-$C_6$-Alkyl, $C_3$-$C_6$-Cycloalkyl, $C_1$-$C_6$-Haloalkyl oder unsubstituiertes oder substituiertes Phenyl, Benzyl, Pyridyl, Pyrimidyl, Pyridazinyl oder Thiazolyl, wobei als Substituenten Halogen, $C_1$-$C_6$-Halogenalkyl, $C_1$-$C_6$-Halogenalkoxy, $C_1$-$C_6$-Alkyl und $C_1$-$C_6$-Alkoxy in Betracht kommen.

In den vorstehenden und folgenden Ausführungen werden unter Alkyl, Alkenyl, Alkinyl und Alkoxy lineare und verzweigtkettige Gruppen verstanden, sofern nicht anders angegeben ist.

Geeignete Phasentransferkatalysatoren sind z.B. Polyether, vorzugsweise Polyethylenglykole, Phosphoniumsalze und insbesondere Ammoniumsalze der Formel III,

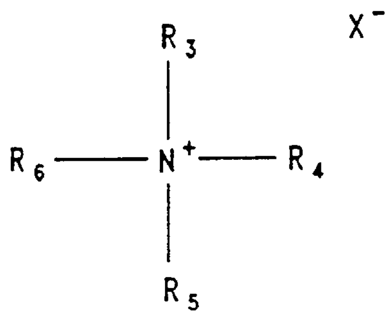

(III)

worin $R^3$ - $R^6$ gleich oder verschieden sind und für Benzyl, Phenyl, ($C_1$-$C_{16}$)-Alkyl, vorzugsweise für ($C_1$-$C_8$)-Alkyl, insbesondere für Butyi, und $X^-$ für $OH^-$ bzw. für Anionen ein- bzw. mehrwertiger Säuren, insbesondere für $Cl^-$, $Br^-$, bzw. $HSO_4^-$ stehen.

Von den erfindungsgemäß zu verwendenden Verbindungen der Struktur III, wobei $R^3$-$R^6$ und $X^-$ die oben genannte Bedeutung besitzen, seien beispielsweise genannt: Benzyltrimethylammoniumchlorid, Tetraethylammoniumbromid, Tetrapropylammoniumbromid, Hexadecyltrimethylammoniumbromid, Benzyltributylammoniumchlorid, vorzugsweise Tetrabutylammoniumbromid bzw. Tetrabutylammoniumhydrogensulfat.

Unter den erfindungsgemäßen Bedingungen werden die Verbindungen der Struktur III zu 0,1 - 20 Mol%, bezogen auf die eingesetzten Verbindungen der Struktur II, vorzugsweise jedoch zu 1 - 10 Mol%, zugesetzt.

Die Verbindungen der Struktur IV werden als solche oder in Form ihrer Salze, insbesondere der Alkali- oder Erdalkalisalze verwendet. Bevorzugte Verbindungen IV sind HOCl, $HClO_4$, HOBr, $HBrO_2$, $HBrO_3$, $HJO_4$ bzw. deren Salze, insbesondere HOCl, HOBr bzw. deren Alkali- oder Erdalkalisalze. Insbesondere sind Natrium-, Kalium-, bzw. Kalziumsalze geeignet, beispielsweise Natriumhypochlorit, Kaliumhypochlorit, Natriumperchlorat, Natriumhypobromit, Natriumbromat, Natriumbromit, Kalziumhypochlorit, vorzugsweise aber Natriumhypochlorit, Natriumhypobromit bzw. Kalziumhypochlorit.

Die Verbindungen der Formel IV sind allgemein bekannte Oxidationsmittel und können als solche bei dem erfindungsgemäßen Verfahren eingesetzt oder durch geeignete Methoden in situ erzeugt werden. Geeignete Methoden sind z.B. die Erzeugung auf elektrochemischem Wege (J. Appl. Elektrochem. (1989), 19, 922) oder die Umsetzung von $H_2O_2$ mit Halogenwasserstoffsäuren bzw. deren Salzen in wäßrigem Milieu (DE-A-21 10 210).

Der Einsatz der Verbindungen der Struktur IV in Form ihrer wäßrigen Lösungen ist bevorzugt.

Bei der Erzeugung der Oxidationsmittel der Struktur IV in situ ist die elektrochemische Erzeugung bevorzugt.

Der Gehalt an IV liegt bei 1 - 20 Gew.-%, vorzugsweise bei 5 - 15 Gew.-%. Bei Erzeugung in situ ist es nicht erforderlich die Konzentration der Verbindungen der Struktur IV zu ermitteln, da die Verbindungen der Struktur IV in Gegenwart der Verbindungen der Struktur II erzeugt werden und mit diesen abreagieren.

Die Menge an Oxidationsmittel IV, welche zur Herstellung der Verbindung der Formel I benötigt wird, liegt bei mindestens 1 Mol pro 1 Mol Edukt II, vorzugsweise bei 1-4 Mol pro Mol Edukt II, insbesondere bei 1-2 Mol pro Mol Alkohol II.

Die erfindungsgemäße Umsetzung der Verbindungen II und IV in Gegenwart eines Phasentransferkatalysators wie z.B. einer Verbindung III erfolgt in einem zweiphasigen System bei Temperaturen von 0°C bis zum Siedepunkt der Mischung.

Die erfindungsgemäße Umsetzung der Verbindungen II und IV in Gegnwart von III erfolgt vorzugsweise im Temperaturbereich von 0 bis 40°C.

Das erfindungsgemäße Verfahren läßt sich zweckmäßig in der Weise durchführen, daß man eine Lösung eines Alkohols der Struktur II bei Raumtemperatur vorlegt und in Gegenwart eines Phasentransferkatalysators der Struktur III eine wässrige Lösung des Oxidationsmittels der Struktur IV unter starkem Rühren zudosiert oder in wäßriger Lösung in situ erzeugt. Nach bis zu weiteren 8 Stunden Reaktionszeit trennt man die Phasen, extrahiert die wässrige Phase mehrmals, wäscht die vereinten organischen Phasen mit gesättigter wässriger Natriumchlorid-Lösung, trocknet die organische Phase mit Natriumsulfat und destilliert die Leichtsieder ab. Das verbleibende Rohprodukt wird im Hochvakuum durch Destillation gereinigt. Dabei fallen die Ketone der Struktur I in hoher Reinheit an.

Die elektrochemische in situ Erzeugung des Oxidationsmittels ist Stand der Technik [J. Appl. Electrochem. 19 (1989) 922]. Sie erfolgt galvanostatisch in ungeteilter Zelle an inerten Elektroden. In der Elektrolysezelle befindet sich eine wäßrige Lösung einer Verbindung YH, worin Y die obengenannte Bedeutung besitzt, oder von deren Salzen in einer Konzentration von 0,001 - 2 Mol/l, bevorzugt von 0,05 bis 0,5 Mol/l, sowie das aprotische Lösemittel zusammen mit dem Phasentransferkatalysator, vorzugsweise mit der Struktur III, und der Verbindung der Struktur II wie beschrieben.

Der pH-Wert der wäßrigen Lösung während der Elektrolyse liegt im Bereich von 5 bis 11, bevorzugt von 6,5 bis 10,5. Die Stromdichte wird im Bereich von 1 bis 100 $mA/cm^2$ gehalten, bevorzugt von 10 bis 40 $mA/cm^2$. Der Stromdurchsatz liegt im Bereich von 1 bis 20 F/Mol, bevorzugt von 2 bis 10 F/Mol.

Die Verbindungen der Formel II, wobei R die oben genannte Bedeutung besitzt, sind aus den entsprechenden Aldehyden und Trifluorbrommethan in Gegenwart von Zink zugänglich (US 4,701,569).

Nachfolgende Beispiele dienen zur näheren Erläuterung der Erfindung:

Beispiel 1

2-Chlor-5-trifluoracetylpyridin

Bei Raumtemperatur werden 21,13 g (0.1 Mol) 2-Chlor-5-(2,2,2-trifluor-1-hydroxyethyl)-pyridin und 1.8 g (0.005 Mol) Tetrabutylammoniumhydrogensulfat in 200 ml Ethylacetat gelöst. Unter starkem Rühren dosiert man innerhalb von 15 Minuten 61 ml (0.12 Mol) einer ca. 12 %igen Natriumhypochlorit-Lösung zu, rührt weitere 2 Stunden, wobei die Reaktionstemperatur auf 40 °C ansteigt, trennt die Phasen, extrahiert die wässrige Phase mehrmals mit Ethylacetat und wäscht die vereinten organischen Phasen mit gesättigter wässriger Natriumchlorid-Lösung. Nach Trocknen der organischen Phase mit Natriumsulfat destilliert man die Leichtsieder ab. Nach Destillation des Rohprodukts bei 0.3 mbar erhält man 15.6 g (74.4 % d. Th.) 2-Chlor-5-trifluoracetylpyridin vom Siedepunkt 50-51 °C, welches laut GC 99.8 %ig ist. $n_D^{20}$: 1.4887.

Beispiel 2

2-Chlor-5-trifluoracetylpyridin

Bei Raumtemperatur werden 2,11 g (0.01 Mol) 2-Chlor-5-(2,2,2-trifluor-1-hydroxyethyl)-pyridin und 0.18 g (0.0005 Mol) Tetrabutylammoniumhydrogensulfat in 30 ml Methylenchlorid gelöst. Unter starkem Rühren dosiert man innerhalb von 5 Minuten 7.3 ml (0.012 Mol) einer ca. 10 %igen Natriumhypochlorit-Lösung zu, rührt weitere 2 Stunden, wobei

die Reaktionstemperatur auf 30 °C ansteigt, gibt die Reaktionsmischung auf 50 ml Wasser, trennt die Phasen, extrahiert die wässrige Phase mehrmals mit Methylenchlorid und wäscht die vereinten organischen Phasen mit gesättigter wässriger Natriumchlorid-Lösung. Nach Trocknen der organischen Phase mit Natriumsulfat destilliert man die Leichtsieder ab. Nach Destillation des Rohprodukts im Kugelrohr (0.5 mbar, 100-120 °C Badtemperatur) erhält man 1.13 g (54 % d. Th.) 2-Chlor-5-trifluoracetylpyridin, welches laut GC 99.8 %ig ist. $n_D^{20}$: 1.4887.

Beispiel 3

2-Chlor-5-trifluoracetylpyridin

Bei Raumtemperatur werden 2,11 g (0.01 Mol) 2-Chlor-5-(2,2,2-trifluor-1-hydroxyethyl)-pyridin und 0.18 g (0.0005 Mol) Tetrabutylammoniumhydrogensulfat in 25 ml Chlorbenzol gelöst. Unter starkem Rühren dosiert man innerhalb von 10 Minuten 12 ml (0.02 Mol) einer ca. 12% igen Natriumhypochlorit-Lösung zu, rührt weitere 4 Stunden, wobei die Reaktionstemperatur auf 30 °C ansteigt, gibt die Reaktionsmischung auf 50 ml Wasser, trennt die Phasen, extrahiert die wässrige Phase mehrmals mit Methylenchlorid und wäscht die vereinten organischen Phasen mit gesättigter wässriger Natriumchlorid-Lösung. Nach Trocknen der organischen Phase mit Natriumsulfat destilliert man die Leichtsieder ab. Nach Destillation des Rohprodukts im Kugelrohr (0.5 mbar, 100-120 °C Badtemperatur) erhält man 1.41 g (67 % d. Th.) 2-Chlor-5-trifluoracetylpyridin, welches laut GC 98 %ig ist.

Beispiel 4

2-Chlor-5-trifluoracetylpyridin

Bei Raumtemperatur werden 2,11 g (0.01 Mol) 2-Chlor-5-(2,2,2-trifluor-1-hydroxyethyl)-pyridin und 0.18 g (0.0005 Mol) Tetrabutylammoniumhydrogensulfat in 30 ml Xylol gelöst. Unter starkem Rühren dosiert man innerhalb von 5 Minuten 7.3 ml (0.012 Mol) einer ca. 10 %igen Natriumhypochlorit-Lösung zu, rührt weitere 2 Stunden, wobei die Reaktionstemperatur auf 28 °C ansteigt, gibt die Reaktionsmischung auf 50 ml Wasser, trennt die Phasen, extrahiert die wässrige Phase mehrmals mit Xylol und wäscht die vereinten organischen Phasen mit gesättigter wässriger Natriumchlorid-Lösung. Nach Trocknen der organischen Phase mit Natriumsulfat destilliert man die Leichtsieder ab. Nach Destillation des Rohprodukts im Kugelrohr (0.4 mbar, 100-110 °C Badtemperatur) erhält man 1.89 g (90 % d. Th.) 2-Chlor-5-trifluoracetylpyridin.

Beispiel 5

2-Chlor-5-trifluoracetylpyridin

Bei Raumtemperatur werden 2,11 g (0.01 Mol) 2-Chlor-5-(2,2,2-trifluor-1-hydroxyethyl)-pyridin und 0.18 g (0.0005 Mol) Tetrabutylammoniumhydrogensulfat in 25 ml Toluol gelöst. Unter starkem Rühren dosiert man innerhalb von 5 Minuten 6.1 ml (0.012 Mol) einer ca. 12 %igen Natriumhypochlorit-Lösung zu, rührt weitere 2.5 Stunden, wobei die Reaktionstemperatur auf 27°C ansteigt, gibt die Reaktionsmischung auf 50 ml Wasser, trennt die Phasen, extrahiert die wässrige Phase mehrmals mit Toluol und wäscht die vereinten organischen Phasen mit gesättigter wässriger Natriumchlorid-Lösung. Nach Trocknen der organischen Phase mit Natriumsulfat destilliert man die Leichtsieder ab. Nach Destillation des Rohprodukts im Kugelrohr (0.4 mbar, 100-115°C Badtemperatur) erhält man 1.78 g (85 % d. Th.) 2-Chlor-5-trifluoracetylpyridin.

Beispiel 6

2-Chlor-5-trifluoracetylpyridin

Bei Raumtemperatur werden 2,11 g (0.01 Mol) 2-Chlor-5-(2,2,2-trifluor-1-hydroxyethyl)-pyridin und 0.17 g (0.0005 Mol) Tetrabutylammoniumbromid in 25 ml Methylenchlorid gelöst. Unter starkem Rühren dosiert man innerhalb von 5 Minuten 12 ml (0.02 Mol) einer ca. 12 %igen Natriumhypochlorit-Lösung zu, rührt weitere 4 Stunden, wobei die Reaktionstemperatur auf 30°C ansteigt, gibt die Reaktionsmischung auf 50 ml Wasser, trennt die Phasen, extrahiert die wässrige Phase mehrmals mit Methylenchlorid und wäscht die vereinten organischen Phasen mit gesättigter wässriger Natriumchlorid-Lösung. Nach Trocknen der organischen Phase mit Natriumsulfat destilliert man die Leichtsieder ab. Nach Destillation des Rohprodukts im Kugelrohr (0.5 mbar, 100-120°C Badtemperatur) erhält man 1.47 g (70.5 % d. Th.) 2-Chlor-5-trifluoracetylpyridin.

Beispiel 7

2-Chlor-5-trifluoracetylpyridin

Bei Raumtemperatur werden 2,11 g (0.01 Mol) 2-Chlor-5-(2,2,2-trifluor-1-hydroxyethyl)-pyridin und 0.18 g (0.0005 Mol) Tetrabutylammoniumhydrogensulfat in 20 ml Methylenchlorid gelöst. Nach Zugabe von 4 ml Wasser und 1.72 g (0.012 Mol) Kalziumhypochlorit wird unter kräftiger Durchmischung 2,25 Stunden gerührt, wobei die Reaktionstemperatur auf 28°C ansteigt. Man gibt die Reaktionsmischung auf 50 ml Wasser, trennt die Phasen, extrahiert die wässrige Phase mehrmals mit Methylenchlorid und wäscht die vereinten organischen Phasen mit gesättigter wässriger Natriumchlorid-Lösung. Nach Trocknen der organischen Phase mit Natriumsulfat destilliert man die Leichtsieder ab. Nach Destillation des Rohprodukts im Kugelrohr (0.4 mbar, 100-110°C Badtemperatur) erhält man 1.69 g (81 % d. Th.) 2-Chlor-5-trifluoracetylpyridin.

Beispiel 8

2-(2,2,2-Trifluorethoxy)-5-trifluoracetylpyridin

Bei Raumtemperatur werden 2,75 g (0.01 Mol) 2-(2,2,2-Trifluorethoxy)-5-(2,2,2-trifluor-1-hydroxyethyl)-pyridin und 0.18 g (0.0005 Mol) Tetrabutylammoniumhydrogensulfat in 30 ml Methylenchlorid gelöst. Unter starkem Rühren dosiert man innerhalb von 10 Minuten 22 ml (0.036 Mol) einer ca. 12 %igen Natriumhypochlorit-Lösung zu, rührt weitere 4.5 Stunden, wobei die Reaktionstemperatur auf 25°C ansteigt, gibt die Reaktionsmischung auf 50 ml Wasser, trennt die Phasen, extrahiert die wässrige Phase mehrmals mit Methylenchlorid und wäscht die vereinten organischen Phasen mit gesättigter wässriger Natriumchlorid-Lösung.

Nach Trocknen der organischen Phase mit Natriumsulfat destilliert man die Leichtsieder ab. Nach Destillation des Rohprodukts im Kugelrohr (0.3 mbar, 100-120°C Badtemperatur) erhält man 2.48 g (90 % d. Th.) 2-(2,2,2-Trifluorethoxy)-5-trifluoracetylpyridin, welches laut GC 96 %ig ist. $n_D^{20}$: 1.4312.

Beispiel 9

2-(2,2,2-Trifluorethoxy)-5-trifluoracetylpyridin

Bei Raumtemperatur werden 43.15 g (0.157 Mol) 2-(2,2,2-Trifluorethoxy)-5-(2,2,2-trifluor-1-hydroxyethyl)-pyridin und 2.66 g (0.0078 Mol) Tetrabutylammoniumhydrogensulfat in 400 ml Ethylacetat gelöst. Unter starkem Rühren dosiert man innerhalb von 1 Stunde 240 ml (0.235 Mol) einer ca. 6 %igen Natriumhypochlorit-Lösung zu, rührt weitere 2 Stunden, wobei die Reaktionstemperatur auf 40°C ansteigt, gibt die Reaktionsmischung auf 500 ml Wasser, trennt die Phasen, extrahiert die wässrige Phase mehrmals mit Ethylacetat und wäscht die vereinten organischen Phasen mit gesättigter wässriger Natriumchlorid-Lösung. Nach Trocknen der organischen Phase mit Natriumsulfat destilliert man die Leichtsieder ab. Nach Destillation des Rohprodukts (27 Torr, 100-102°C) erhält man 24.47 g (57 % d. Th.) 2-(2,2,2-Trifluorethoxy)-5-trifluoracetylpyridin, welches laut GC 97 %ig ist.

Beispiel 10

2-(2,2,2-Trifluorethoxy)-5-trifluoracetylpyridin

Bei Raumtemperatur werden 2,75 g (0.01 Mol) 2-(2,2,2-Trifluorethoxy)-5-(2,2,2-trifluor-1-hydroxyethyl)-pyridin und 0.18 g (0.0005 Mol) Tetrabutylammoniumhydrogensulfat in 25 ml Chlorbenzol gelöst. Unter starkem Rühren dosiert man innerhalb von 10 Minuten 12.2 ml (0.012 Mol) einer ca. 6 %igen Natriumhypochlorit-Lösung zu, rührt weitere 8 Stunden, wobei die Reaktionstemperatur auf 24°C ansteigt, gibt die Reaktionsmischung auf 50 ml Wasser, trennt die Phasen, extrahiert die wässrige Phase mehrmals mit Methylenchlorid und wäscht die vereinten organischen Phasen mit gesättigter wässriger Natriumchlorid-Lösung. Nach Trocknen der organischen Phase mit Natriumsulfat destilliert man die Leichtsieder ab. Nach Destillation des Rohprodukts im Kugelrohr (0.3 mbar, 100-120°C Badtemperatur) erhält man 2.0 g (73.3 % d. Th.) 2-(2,2,2-Trifluorethoxy)-5-trifluoracetylpyridin.

Beispiel 11

2- (2,2,2-Trifluorethoxy)-5-trifluoracetylpyridin

Bei Raumtemperatur werden 2,75 g (0.01 Mol) 2-(2,2,2-Trifluorethoxy)-5-(2,2,2-trifluor-1-hydroxyethyl)-pyridin und

0.18 g (0.0005 Mol) Tetrabutylammoniumhydrogensulfat in 30 ml Xylol gelöst. Unter starkem Rühren dosiert man innerhalb von 5 Minuten 6.1 ml (0.012 Mol) einer ca. 12 %igen Natriumhypochlorit-Lösung zu, rührt weitere 4 Stunden, wobei die Reaktionstemperatur auf 28°C ansteigt, gibt die Reaktionsmischung auf 50 ml Wasser, trennt die Phasen, extrahiert die wässrige Phase mehrmals mit Xylol und wäscht die vereinten organischen Phasen mit gesättigter wässriger Natriumchlorid-Lösung. Nach Trocknen der organischen Phase mit Natriumsulfat destilliert man die Leichtsieder ab. Nach Destillation des Rohprodukts im Kugelrohr (0.3 mbar, 100-125°C Badtemperatur) erhält man 2.31 g (84.7 % d. Th.) 2-(2,2,2-Trifluorethoxy)-5-trifluoracetylpyridin.

Beispiel 12

2-(2,2,2-Trifluorethoxy)-5-trifluoracetylpyridin

Bei Raumtemperatur werden 2,75 g (0.01 Mol) 2-(2,2,2-Trifluorethoxy)-5-(2,2,2-trifluor-1-hydroxyethyl)-pyridin und 0.18 g (0.0005 Mol) Tetrabutylammoniumhydrogensulfat in 20 ml Methylenchlorid gelöst. Nach Zugabe von 4 ml Wasser und 1.72 g (0.012 Mol) Kalziumhypochlorit wird unter kräftiger Durchmischung 4 Stunden gerührt, wobei die Reaktionstemperatur auf 25°C ansteigt. Man gibt die Reaktionsmischung auf 50 ml Wasser, trennt die Phasen, extrahiert die wässrige Phase mehrmals mit Methylenchlorid und wäscht die vereinten organischen Phasen mit gesättigter wässriger Natriumchlorid-Lösung. Nach Trocknen der organischen Phase mit Natriumsulfat destilliert man die Leichtsieder ab. Nach Destillation des Rohprodukts im Kugelrohr (0.3 mbar, 100-120°C Badtemperatur) erhält man 2.38 g (87 % d. Th.) 2-(2,2,2-Trifluorethoxy)-5-trifluoracetylpyridin.

Beispiel 13

2-(2,2,2-Trifluorethoxy)-5-trifluoracetylpyridin

Bei Raumtemperatur werden 2,75 g (0.01 Mol) 2-(2,2,2-Trifluorethoxy)-5-(2,2,2-trifluor-1-hydroxyethyl)-pyridin und 0.17 g (0.0005 Mol) Tetrabutylammoniumbromid in 25 ml Methylenchlorid gelöst. Unter starkem Rühren dosiert man innerhalb von 10 Minuten 12.2 ml (0.012 Mol) einer ca. 6 %igen Natriumhypochlorit-Lösung zu, rührt weitere 8 Stunden, wobei die Reaktionstemperatur auf 27°C ansteigt, gibt die Reaktionsmischung auf 50 ml Wasser, trennt die Phasen, extrahiert die wässrige Phase mehrmals mit Methylenchlorid und wäscht die vereinten organischen Phasen mit gesättigter wässriger Natriumchlorid-Lösung. Nach Trocknen der organischen Phase mit Natriumsulfat destilliert man die Leichtsieder ab. Nach Destillation des Rohprodukts im Kugelrohr (0.3 mbar, 100-120°C Badtemperatur) erhält man 1.31 g (48 % d. Th.) 2-(2,2,2-Trifluorethoxy)-5-trifluoracetylpyridin.

Beispiel 14

2-(2,2,2-Trifluorethoxy)-5-trifluoracetylpyridin

Bei Raumtemperatur werden 2,75 g (0.01 Mol) 2-(2,2,2-Trifluorethoxy)-5-(2,2,2-trifluor-1-hydroxyethyl)-pyridin und 0.17 g (0.0005 Mol) Tetrabutylammoniumbromid in 30 ml Chlorbenzol gelöst. Unter starkem Rühren dosiert man innerhalb von 5 Minuten 6.1 ml (0.012 Mol) einer ca. 12 %igen Natriumhypochlorit-Lösung zu, rührt weitere 4 Stunden bei Raumtemperatur, gibt die Reaktionsmischung auf 50 ml Wasser, trennt die Phasen, extrahiert die wässrige Phase mehrmals mit Methylenchlorid und wäscht die vereinten organischen Phasen mit gesättigter wässriger Natriumchlorid-Lösung. Nach Trocknen der organischen Phase mit Natriumsulfat destilliert man die Leichtsieder ab. Nach Destillation des Rohprodukts im Kugelrohr (0.3 mbar, 100-120°C Badtemperatur) erhält man 0.68 g (24.7 % d. Th.) 2-(2,2,2-Trifluorethoxy)-5-trifluoracetylpyridin.

Beispiel 15

4-Trifluoracetyl-brombenzol

Bei Raumtemperatur werden 6.88 g (0.027 Mol) 2,2,2-Trifluor-1-(4-bromphenyl)ethanol und 0.46 g (0.00135 Mol) Tetrabutylammoniumhydrogensulfat in 100 ml Methylenchlorid gelöst. Unter starkem Rühren dosiert man innerhalb von 15 Minuten 16.6 ml (0.032 Mol) einer ca. 12 %igen Natriumhypochlorit-Lösung zu, rührt weitere 6 Stunden, wobei die Reaktionstemperatur auf 28°C ansteigt, gibt die Reaktionsmischung auf 100 ml Wasser, trennt die Phasen, extrahiert die wässrige Phase mehrmals mit Methylenchlorid und wäscht die vereinten organischen Phasen mit gesättigter wässriger Natriumchlorid-Lösung. Nach Trocknen der organischen Phase mit Natriumsulfat destilliert man die Leichtsieder ab. Nach Destillation des Rohprodukts im Kugelrohr (0.5 mbar, 90-110°C Badtemperatur) erhält man 4.13 g

(60.4 % d. Th.) 4-Trifluoracetyl-brombenzol. $n_D^{20}$: 1.5112

Beispiel 16

3-Trifluoracetyl-diphenylether

Bei Raumtemperatur werden 3.34 g (0.0125 Mol) 2,2,2-Trifluor-1-(3-phenoxyphenyl)ethanol und 0.21 g (0.0006 Mol) Tetrabutylammoniumhydrogensulfat in 75 ml Methylenchlorid gelöst. Unter starkem Rühren dosiert man innerhalb von 10 Minuten 7.7 ml (0.015 Mol) einer ca. 12 %igen Natriumhypochlorit-Lösung zu, rührt weitere 6 Stunden, wobei die Reaktionstemperatur auf 27°C ansteigt, gibt die Reaktionsmischung auf 100 ml Wasser, trennt die Phasen, extrahiert die wässrige Phase mehrmals mit Methylenchlorid und wäscht die vereinten organischen Phasen mit gesättigter wässriger Natriumchlorid-Lösung. Nach Trocknen der organischen Phase mit Natriumsulfat destilliert man die Leichtsieder ab. Nach Destillation des Rohprodukts im Kugelrohr (0.5 mbar, 150-180°C Badtemperatur) erhält man 2.09 g (62.7 % d. Th.) 3-Trifluoracetyl-diphenylether. $n_D^{20}$: 1.5348.

Beispiel 17

3-Trifluoracetylanisol

Bei Raumtemperatur werden 4.82 g (0.023 Mol) 2,2,2-trifluor-1-(3-methoxyphenyl)ethanol und 0.4 g (0.0012 Mol) Tetrabutylammoniumhydrogensulfat in 100 ml Methylenchlorid gelöst. Unter starkem Rühren dosiert man innerhalb von 15 Minuten 14.4 ml (0.028 Mol) einer ca. 12 %igen Natriumhypochlorit-Lösung zu, rührt weitere 6 Stunden, wobei die Reaktionstemperatur auf 26°C ansteigt, gibt die Reaktionsmischung auf 100 ml Wasser, trennt die Phasen, extrahiert die wässrige Phase mehrmals mit Methylenchlorid und wäscht die vereinten organischen Phasen mit gesättigter wässriger Natriumchlorid-Lösung. Nach Trocknen der organischen Phase mit Natriumsulfat destilliert man die Leichtsieder ab. Nach Destillation des Rohprodukts im Kugelrohr (0.2 mbar, 60-70°C Badtemperatur) erhält man 2.1 g (44 % d. Th.) 3-Trifluoracetylanisol. $n_D^{20}$: 1.4773

Beispiel 18

2-Brom-4-Trifluoracetyl-fluorbenzol

Bei Raumtemperatur werden 6.4 g (0.023 Mol) 2,2,2-trifluor-1-(3-brom-4-fluorphenyl)-ethanol und 0.4 g (0.0012 Mol) Tetrabutylammoniumhydrogensulfat in 100 ml Methylenchlorid gelöst. Unter starkem Rühren dosiert man innerhalb von 15 Minuten 14.4 ml (0.028 Mol) einer ca. 12 %igen Natriumhypochlorit-Lösung zu, rührt weitere 4 Stunden, wobei die Reaktionstemperatur auf 27°C ansteigt, gibt die Reaktionsmischung auf 100 ml Wasser, trennt die Phasen, extrahiert die wässrige Phase mehrmals mit Methylenchlorid und wäscht die vereinten organischen Phasen mit gesättigter wässriger Natriumchlorid-Lösung. Nach Trocknen der organischen Phase mit Natriumsulfat destilliert man die Leichtsieder ab. Nach Destillation des Rohprodukts im Kugelrohr (0.5 mbar, 80-100°C Badtemperatur) erhält man 3.9 g (61.5 % d. Th.) 2-Brom-4-Trifluoracetyl-fluorbenzol. $n_D^{20}$: 1.4905.

Beispiel 19

4-Trifluoracetyl-methylbenzol

Bei Raumtemperatur werden 2.88 g (0.015 Mol) 2,2,2-trifluor-1-(4-methylphenyl)ethanol und 0.26 g (0.0007 Mol) Tetrabutylammoniumhydrogensulfat in 125 ml Methylenchlorid gelöst. Unter starkem Rühren dosiert man innerhalb von 10 Minuten 9.3 ml (0.018 Mol) einer ca. 12 %igen Natriumhypochlorit-Lösung zu, rührt weitere 4 Stunden bei Raumtemperatur, gibt die Reaktionsmischung auf 100 ml Wasser, trennt die Phasen, extrahiert die wässrige Phase mehrmals mit Methylenchlorid und wäscht die vereinten organischen Phasen mit gesättigter wässriger Natriumchlorid-Lösung. Nach Trocknen der organischen Phase mit Natriumsulfat destilliert man die Leichtsieder ab. Nach Destillation des Rohprodukts im Kugelrohr (0.4 mbar, 60-70°C Badtemperatur) erhält man 0.84 g (30 % d. Th.) 4-Trifluoracetyl-methylbenzol. $n_D^{20}$: 1.4675.

Beispiel 20

5-Brom-2-trifluoracetylthiophen

Bei Raumtemperatur werden 2.93 g (0.011 Mol) 2,2,2-trifluor-1-(5-brom-2-thienyl)ethanol und 0.19 g (0.0006 Mol) Tetrabutylammoniumhydrogensulfat in 100 ml Methylenchlorid gelöst. Unter starkem Rühren dosiert man innerhalb von 5 Minuten 6.9 ml (0.014 Mol) einer ca. 12 %igen Natriumhypochlorit-Lösung zu, rührt weitere 4 Stunden bei raumtemperatur, gibt die Reaktionsmischung auf 100 ml Wasser, trennt die Phasen, extrahiert die wässrige Phase mehrmals mit Methylenchlorid und wäscht die vereinten organischen Phasen mit gesättigter wässriger Natriumchlorid-Lösung. Nach Trocknen der organischen Phase mit Natriumsulfat destilliert man die Leichtsieder ab. Nach Destillation des Rohprodukts im Kugelrohr (0.3 mbar, 40-60°C Badtemperatur) erhält man 0.48 g (39 % d. Th.) 5-Brom-2-trifluoracetylthiophen, $n_D^{20}$: 1.5309.

Beispiel 21

3-Trifluoracetylpyridin

Bei Raumtemperatur werden 13.2 g (0.075 Mol) 2,2,2-trifluor-1-(3-pyridyl)-ethanol und 1.26 g (0.0037Mol) Tetrabutylammoniumhydrogensulfat in 250 ml Methylenchlorid gelöst. Unter starkem Rühren dosiert man innerhalb von 20 Minuten 46 ml (0.09 Mol) einer ca. 12 %igen Natriumhypochlorit-Lösung zu, rührt weitere 4 Stunden, wobei die Reaktionstemperatur auf 35°C steigt, gibt die Reaktionsmischung auf 250 ml Wasser, trennt die Phasen, extrahiert die wässrige Phase mehrmals mit Methylenchlorid und wäscht die vereinten organischen Phasen mit gesättigter wässriger Natriumchlorid-Lösung. Nach Trocknen der organischen Phase mit Natriumsulfat destilliert man die Leichtsieder ab. Nach Destillation des Rohprodukts (28 Torr, 65-70°C) erhält man 9.39 g (72 % d.Th.) 3-Trifluoracetylpyridin. $n_d^{20}$: 1.4796.

Beispiel 22

2-Phenoxy-4-trifluoracetyl-fluorbenzol

Bei Raumtemperatur werden 14.37 g (0.05 Mol) 2,2,2-trifluor-1-(4-fluor-3-phenoxyphenyl)-ethanol und 0.85 g (0.0025 Mol) Tetrabutylammoniumhydrogensulfat in 200 ml Methylenchlorid gelöst. Unter starkem Rühren dosiert man innerhalb von 20 Minuten 31 ml (0.06 Mol) einer ca. 12 %igen Natriumhypochlorit-Lösung zu, rührt weitere 6 Stunden bei Raumtemperatur, gibt die Reaktionsmischung auf 200 ml Wasser, trennt die Phasen, extrahiert die wässrige Phase mehrmals mit Methylenchlorid und wäscht die vereinten organischen Phasen mit gesättigter wässriger Natriumchlorid-Lösung. Nach Trocknen der organischen Phase mit Natriumsulfat destilliert man die Leichtsieder ab. Nach Destillation des Rohprodukts im Kugelrohr (0.2 mbar, 150-165°C Badtemperatur) erhält man 10.8 g (76 % d. Th.) 2-Phenoxy-4-trifluoracetyl-fluorbenzol.
$n_D^{20}$: 1.5230.

Beispiel 23

Trifluoracetylbenzol

Bei Raumtemperatur werden 10.0 g (0.057 Mol) 2,2,2-trifluor-1-phenyl-ethanol und 0.96 g (0.0028 Mol) Tetrabutylammoniumhydrogensulfat in 250 ml Methylenchlorid gelöst. Unter starkem Rühren dosiert man innerhalb von 20 Minuten 35 ml (0.068 Mol) einer ca. 12 %igen Natriumhypochlorit-Lösung zu, rührt weitere 6 Stunden bei Raumtemperatur, gibt die Reaktionsmischung auf 200 ml Wasser, trennt die Phasen, extrahiert die wässrige Phase mehrmals mit Methylenchlorid und wäscht die vereinten organischen Phasen mit gesättigter wässriger Natriumchlorid-Lösung. Nach Trocknen der organischen Phase mit Natriumsulfat destilliert man die Leichtsieder ab. Nach Destillation des Rohprodukts im Kugelrohr (24 Torr, 90-115°C Badtemperatur) erhält man 7.45 g (75 % d. Th.) Trifluoracetylbenzol. $n_D^{20}$: 1.4658.

Beispiel 24

4-Trifluoracetyl-trifluormethylbenzol

Bei Raumtemperatur werden 25.4 g (0.1 Mol) 2,2,2-trifluor-1-(4-trifluormethylphenyl)ethanol und 1.76 g (0.005

Mol) Tetrabutylammoniumhydrogensulfat in 350 ml Methylenchlorid gelöst. Unter starkem Rühren dosiert man innerhalb von 20 Minuten 108 ml (0.125 Mol) einer ca. 12 %igen Natriumhypochlorit-Lösung zu, rührt weitere 5 Stunden bei Raumtemperatur, gibt die Reaktionsmischung auf 400 ml Wasser, trennt die Phasen, extrahiert die wässrige Phase mehrmals mit Methylenchlorid und wäscht die vereinten organischen Phasen mit gesättigter wässriger Natriumchlorid-Lösung. Nach Trocknen der organischen Phase mit Natriumsulfat destilliert man die Leichtsieder ab. Nach Destillation des Rohprodukts (18 Torr, 60-61°C ) erhält man 16.74 g (66.5 % d. Th.) 4-Trifluoracetyl-trifluormethylbenzol, welches laut GC 96,7 %ig ist. $n_D^{20}$: 1.4146.

Beispiel 25

Trifluormethyl-cyclohexylketon

Bei Raumtemperatur werden 10.67 g (0.059 Mol) 2,2,2-trifluor-1-cyclohexylethanol und 0.98 g (0.003 Mol) Tetrabutylammoniumhydrogensulfat in 250 ml Methylenchlorid gelöst. Unter starkem Rühren dosiert man innerhalb von 20 Minuten 37 ml (0.069 Mol) einer ca. 12 %igen Natriumhypochlorit-Lösung zu, rührt weitere 4 Stunden, wobei die Reaktionstemperatur auf 30°C ansteigt, gibt die Reaktionsmischung auf 200 ml Wasser, trennt die Phasen, extrahiert die wässrige Phase mehrmals mit Methylenchlorid und wäscht die vereinten organischen Phasen mit gesättigter wässriger Natriumchlorid-Lösung. Nach Trocknen der organischen Phase mit Natriumsulfat destilliert man die Leichtsieder ab. Nach Destillation des Rohprodukts (28 Torr, 80-82°C) erhält man 4.86 g (46 % d. Th.) Trifluormethylcyclohexylketon. $n_D^{20}$: 1.4043.

Beispiel 26

6-Phenoxy-2-trifluoracetylpyridin

Bei Raumtemperatur werden 9.38 g (0.035 Mol) 2,2,2-trifluor-1-(6-phenoxy-2-pyridyl)-ethanol und 0.58 g (0.0017 Mol) Tetrabutylammoniumhydrogensulfat in 200 ml Methylenchlorid gelöst. Unter starkem Rühren dosiert man innerhalb von 15 Minuten 22 ml (0.042 Mol) einer ca. 12 %igen Natriumhypochlorit-Lösung zu, rührt weitere 4 Stunden, wobei die Reaktionstemperatur auf 30°C ansteigt, gibt die Reaktionsmischung auf 200 ml Wasser, trennt die Phasen, extrahiert die wässrige Phase mehrmals mit Methylenchlorid und wäscht die vereinten organischen Phasen mit gesättigter wässriger Natriumchlorid-Lösung. Nach Trocknen der organischen Phase mit Natriumsulfat destilliert man die Leichtsieder ab. Nach Destillation des Rohprodukts im Kugelrohr (0.4 mbar, 180°C Badtemperatur) erhält man 4.75 g (50.6 % d. Th.) 6-Phenoxy-2-trifluoracetylpyridin. $n_D^{20}$: 1.5298.

Beispiel 27

2-Chlor-5-trifluoracetylpyridin

Zu einer Mischung aus 200 ml einer 0.2 M NaBr-Lösung und 21,2 g (0,1 Mol) 2-Chlor-5-(2,2,2-trifluor-1-hydroxyethyl)-pyridin, gelöst in 50 ml Ethylacetat, werden 1,7 g (0.005 Mol) Tetrabutylammoniumhydrogensulfat zugegeben. Die wäßrige Phase wird mit verdünnter NaOH-Lösung auf pH 10 gebracht. Diese Mischung wird in ungeteilte Zelle an einer Platin-Anode und einer Edelstahl-Kathode bei Raumtemperatur und einer Stromdichte von 30 $mA/cm^2$ bis zu einem Stromdurchsatz von 21 Ah (7,8 F/Mol) elektrolysiert. GC-Kontrolle zeigt vollständigen Umsatz an, der Anteil an 2-Chlor-5-trifluoracetylpyridin beträgt 72 %. Nach der Elektrolyse werden die Phasen getrennt, die wäßrige Phase wird neutralisiert und mehrmals mit Ethylacetat extrahiert. Die vereinigten organischen Phasen werden mit Natriumsulfat getrocknet und die Leichtsider werden abdestilliert. Nach Destillation des Rohprodukts bei 0,3 mbar erhält man 12,8 g (61,1 % d.Th.) 2-Chlor-5-trifluoracetylpyridin vom Siedepunkt 50 - 51°C, welches laut GC 98,7 %ig ist.

Beispiel 28

2- (2,2,2-Trifluorethoxy)-5-trifluoracetylpyridin

Bei Raumtemperatur werden 2,6 g (0.0095 Mol) 2-(2,2,2-Trifluorethoxy)-5-(2,2,2-trifluor-1-hydroxyethyl)-pyridin und 0,18 g (0.0005 Mol) Tetrabutylammoniumhydrogensulfat in 10 ml Chloroform gelöst und mit 1,07 ml einer 48 %igen, wäßrigen Lösung von HBr versetzt. Unter starkem Rühren tropft man bei ca. 45°C langsam 4,24 ml (0.041 Mol) einer 30 %igen Lösung von $H_2O_2$ zu und läßt weitere 24 Stunden rühren. Man gibt die Reaktionsmischung auf 200 ml Wasser, trennt die Phasen, extrahiert die wäßrige Phase mehrmals mit Chloroform und wäscht die vereinten organischen Phasen mit 5 %iger Natriumhydrogencarbonat-Lösung und mit Wasser. Nach Trocknen der organischen Phase mit Natriumsulfat destilliert man die Leichtsieder ab. Die Destillation des Rohproduktes im Kugelrohr (25 Torr, 100 - 200°C Badtemperatur) liefert 1,65 g (60 % d.Th.) 2-(2,2,2-Trifluorethoxy)-5-trifluoracetylpyridin, welches laut GC 98,3 %ig ist.

Beispiel 29

4-Trifluoracetyl-trifluormethoxybenzol

Bei Raumtemperatur werden 24,0 g (0.09 Mol) 2,2,2-Trifluor-1-(4-trifluormethoxyphenyl)-ethanol und 1,57 g (0.0046 Mol) Tetrabutylammoniumhydrogensulfat in 250 ml Methylenchlorid gelöst. Unter starkem Rühren dosiert man innerhalb von 20 Minuten 100 ml (0.12 Mol) einer ca. 12 %igen Natriumhypochlorit-Lösung zu, rührt weitere 5 Stunden, wobei die Temperatur auf 40°C ansteigt, gibt die Reaktionsmischung auf 400 ml Wasser, trennt die Phasen, extrahiert die wäßrige Phase mehrmals mit Methylenchlorid und wäscht die vereinten organischen Phasen mit gesättigter wäßriger Natriumchlorid-Lösung. Nach Trocknen der organischen Phase mit Natriumsulfat destilliert man die Leichtsieder ab. Nach Destillation des Rohproduktes im Kugelrohr (14 Torr, Badtemperatur 100 - 115°C) erhält man 17,19 g (74 % d. Th.) 4-Trifluoracetyltrifluormethoxybenzol. $n_D^{20}$: 1.4188.

Beispiel 30

4-Trifluoracetyl-difluormethoxybenzol

Bei Raumtemperatur werden 23,67 g (0.098 Mol) 2,2,2-Trifluor-1-(4-difluormethoxyphenyl)-ethanol und 1,66 g (0.0049 Mol) Tetrabutylammoniumhydrogensulfat in 200 ml Methylenchlorid gelöst. Unter starkem Rühren dosiert man innerhalb von 25 Minuten 105 ml (0.124 Mol) einer ca. 12 %igen Natriumhypochlorit-Lösung zu, rührt weitere 5 Stunden, wobei die Temperatur auf 40°C ansteigt, gibt die Reaktionsmischung auf 500 ml Wasser, trennt die Phasen, extrahiert die wäßrige Phase mehrmals mit Methylenchlorid und wäscht die vereinten organischen Phasen mit gesättigter wässriger Natriumchlorid-Lösung. Nach Trocknen der organischen Phase mit Natriumsulfat destilliert man die Leichtsieder ab. Nach Destillation des Rohproduktes im Kugelrohr (15 Torr, Badtemperatur 90 - 110°C) erhält man 16,0 g (68 % d. Th.) 4-Trifluoracetyldifluormethoxybenzol. $n_D^{20}$: 1.4201.

**Patentansprüche**

1. Verfahren zur Herstellung von Verbindungen der Formel I

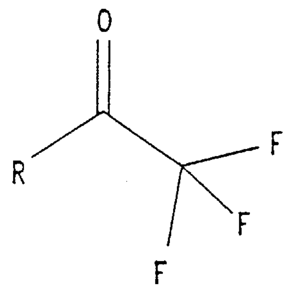

(I)

worin R ein unsubstituierter oder substituierter aliphatischer oder aromatischer Kohlenwasserstoffrest oder ein unsubstituierter oder substituierter aliphatisch- oder aromatisch-heterocyclischer Rest ist, dadurch gekennzeichnet, daß man einen Alkohol der Formel II,

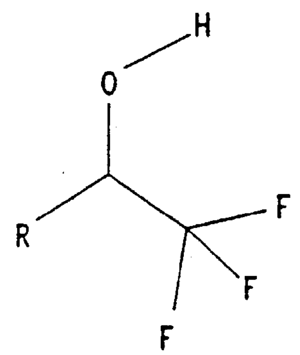

(II)

worin R die oben genannte Bedeutung besitzt, in einem aprotischen Lösemittel, in Gegenwart von 0,1 - 20 Mol.-%, bezogen auf den eingesetzten Alkohol der Struktur II, eines Phasentransferkatalysators mit einer 1 - 20 gew.-%igen wäßrigen Lösung von Verbindungen der Struktur IV oder einer 1 - 20 gew.-%igen wäßrigen Lösung einer in situ erzeugten Verbindung der Struktur IV

$$YO_nH \qquad (IV)$$

worin Y = Cl, Br oder J und n = eine ganze Zahl von 1 bis 4 bedeuten, oder von deren Salzen bei Temperaturen von 0°C bis zum Siedepunkt der Mischung, umsetzt.

**2.** Verfahren gemäß Anspruch 1, dadurch gekennzeichnet, daß man eine Verbindung der Formel IV oder eines ihrer Salze als solche(s) in Form der wäßrigen Lösung einsetzt.

**3.** Verfahren gemäß Anspruch 1 oder 2, dadurch gekennzeichnet, daß man eine wäßrige Lösung einer auf elektrochemischen oder durch Umsetzung von $H_2O_2$ mit Halogenwasserstoffsäure oder deren Salzen in situ hergestellten Verbindung der Formel IV einsetzt.

**4.** Verfahren gemäß einem oder mehreren der Ansprüche 1 bis 3, dadurch gekennzeichnet, daß eine oder mehrere der folgenden Bedingungen eingehalten werden:

a) das aprotische Lösungsmittel Methylenchlorid, Chlorform, Ethylacetat, Toluol, Xylol oder Chlorbenzol ist,
b) der Phasentransferkatalysator ein Ammoniumsalz der Formel III

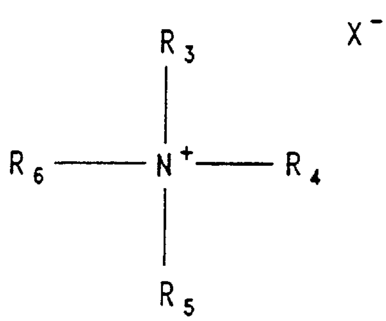

(III)

worin $R^3$ - $R^6$ gleich oder verschieden sind und für Benzyl, Phenyl, $(C_1$-$C_{16})$-Alkyl und $X^-$ für $OH^-$ oder für Anionen ein- bzw. mehrwertiger Säuren, stehen, ist,
c) 1 - 10 Mol.-% Phasentransfertkatalysator bezogen auf den eingesetzten Alkohol II zugesetzt werden,
d) eine 5 - 15 %ige (Gewichtsprozent) wäßrige Lösung einer Verbindung der Formel IV oder eines Alkali- oder Erdalkalisalzes dieser Verbindung verwendet wird,
e) pro Mol Alkohol der Struktur II 1 - 2 Mol Oxidationsmittel der Struktur IV eingesetzt werden,
f) die Reaktionstemperatur 0 bis 40°C beträgt.

**5.** Verfahren gemäß einem oder mehreren der Ansprüche 1 bis 4, dadurch gekennzeichnet, daß eine oder mehrere der folgenden Bedingungen eingehalten werden:

a) das aprotische Lösungsmittel Methylenchlorid, Chloroform, Ethylacetat, Toluol, Xylol oder Chlorbenzol ist,
b) der Phasentransferkatalysator ein Ammoniumsalz der Formel III

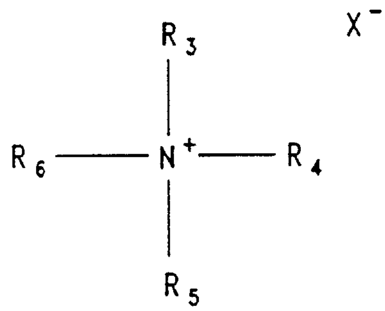

(III)

worin $R^3$ - $R^6$ gleich oder verschieden sind und für Benzyl, Phenyl, $(C_1\text{-}C_8)$-Alkyl und $X^-$ für $Cl^-$, $Br^-$ oder $HSO_4^-$ stehen,
c) 1 - 10 Mol.-% Ammoniumsalz der Formel III als Phasentransferkatalysator zugesetzt werden,
d) als 5 - 15 %ige wäßrige Lösung einer Verbindung der Struktur IV eine wäßrige Lösung von HOCl, $HClO_4$, HOBr, $HBrO_2$, $HBrO_3$, $HJO_4$ oder deren Salzen Verwendung findet,
e) pro Mol Alkohol der Struktur II 1 - 2 Mol Oxidationsmittel der Struktur IV eingesetzt werden,
f) die Reaktionstemperatur 0°C bis 40°C beträgt.

**6.** Verfahren gemäß einem oder mehreren der Ansprüche 1 bis 5, dadurch gekennzeichnet, daß eine oder mehrere der folgenden Bedingungen eingehalten werden:

a) das aprotische Lösungsmittel Methylenchlorid, Ethylacetat, Toluol, Xylol oder Chlorbenzol ist,
b) der Phasentransferkatalisator Benzyltrimethylammoniumchlorid, Tetraethylammoniumbromid, Tetrapropylammoniumbromid, Tetrabutylammoniumbromid, Tetrabutylammoniumdydrogensulfat, Hexadecyltrimethylammoniumbromid oder Benzyltributylammoniumbromid,
c) 1 - 10 Mol.-% Ammoniumsalz der Formel III als Phasentransferkatalisator zugesetzt werden,
d) als 5 - 15 %ige wäßrige Lösung einer Verbindung der Struktur IV eine wäßrige Lösung von HOCl, HOBr, NaOCl, NaOBr oder $Ca(OCl)_2$ verwendet wird,
e) pro Mol Alkohol der Struktur II 1- 2 Mol Oxidationsmittel der Struktur IV eingesetzt werden,
f) die Reaktionstemperatur 0 bis 40°C beträgt.

**7.** Verfahren gemäß einem oder mehreren der Ansprüche 1 bis 6, dadurch gekennzeichnet, daß eine oder mehrere der folgenden Bedingungen eingehalten werden:

a) das aprotische Lösungsmittel Methylenchlorid, Ethylacetat, Toluol, Xylol oder Chlorbenzol ist,
b) der Phasentransferkatalysator Tetrabutylammoniumbromid oder Tetrabutylammoniumhydrogensulfat ist,
c) 1 - 10 Mol.-% Ammoniumsalz der Formel III als Phasentransferkatalysator zugesetzt werden,
d) als 5 - 15 %ige wäßrige Lösung einer Verbindung der Struktur IV eine wäßrige Lösung von Natriumhypobromit Natriumhypochlorit oder Kalziumhypochlorit verwendet wird,
e) pro Mol Alkohol der Struktur II 1 - 2 Mol Oxidationsmittel der Struktur IV eingesetzt werden,
f) die Reaktionstemperatur 0 bis 40°C beträgt.

**8.** Verfahren gemäß einem oder mehreren der Ansprücne 1 bis 7, dadurch gekennzeichnet, daß R in Formel II als Kohlenwasserstoffrest 1 bis 30 C-Atome enthält und als heterocyclischer Rest 4 bis 8 Ringglieder und 1 bis 4 Heteroatome aufweist.

**9.** Verfahren gemäß einem oder mehreren der Ansprüche 1 bis 8, dadurch gekennzeichnet, daß R in Formel II als aliphatischer Kohlenwasserstoffrest unsubstituiertes oder substituiertes Alkyl, Alkenyl, Alkinyl, Cycloalkyl oder Cycloalkenyl, als unsubstituierter oder substituierter aromatischer Kohlenwasserstoffrest Aryl, Aralkenyl, Aralkinyl oder Aralkyl, als unsubstituierter oder substituierter aliphatisch-heterocyclischer Rest ein 3- bis 7-gliedriger, 1 bis

3 Heteroatome enthaltender heterocyclischer Ring und als aromatisch-heterocyclischer Rest ein 5- oder 6-gliedriger, 1 bis 4 Heteroatome enthaltender heterocyclischer Ring ist.

**10.** Verfahren gemäß einem oder mehreren der Ansprüche 1 bis 9, dadurch gekennzeichnet, daß R in Formel II substituiert ist mit $C_1$-$C_{18}$-Alkyl, $C_2$-$C_{18}$-Alkenyl, $C_2$-$C_{18}$-Alkinyl, $C_6$-$C_{18}$-Aryl, $C_7$-$C_{18}$-Aralkyl, $C_7$-$C_{18}$-Alkaryl, $C_8$-$C_{18}$Alkarylalkyl, $C_1$-$C_{18}$-Alkoxy, $C_1$-$C_{18}$-Alkylthio, $C_6$-$C_{18}$-Aryloxy, $C_7$-$C_{18}$-Aralkoxy, $C_7$-$C_{18}$-Aralkylthio, $C_7$-$C_{18}$-Alkaryloxy, $C_8$-$C_{18}$-Alkaralkoxy, $C_7$-$C_{18}$-Aryloxyalkyl, $C_7$-$C_{18}$-Alkoxyaryl, Hetaryl, Hetarylalkyl, Alkylhetaryl, Alkylhetarylalkyl, Hetaryloxy, Hetarylalkyloxy, Hetarylalkylthio, Alkylhetaryloxy, Alkoxyhetaryl, Alkylhetarylalkyloxy, Hetaryloxyalkyl, wobei die heterocyclischen Arylreste jeweils bis zu 8 Kohlenstoffatome und bis zu 4 Heteroatome ausgewählt aus der Gruppe bestehend aus N, S und O, aufweisen und die gegebenenfalls anwesenden Alkylgruppen insgesamt bis zu 12 Kohlenstoffatome besitzen, $C_3$-$C_{12}$-Cycloalkyl, $C_3$-$C_{12}$-Cycloalkoxy, $C_3$-$C_{12}$-Cycloalkenyl, $C_3$-$C_{12}$-Cycloalkenyloxy, $C_1$-$C_{18}$-Haloalkyl, insbesondere Fluoralkyl, -CN, OH, $NO_2$, F, Cl, Br, J, -NCO, $COOR^1$, $COR^1$, $-OCOR^1$, $-CONR^1R^2$, $-NR^1COR^2$, $-NR^1R^2$, $-SO_2NR^1R^2$, $-Si(R^1)_3$, $-SO_2R^1$, $-SO_3R^1$, $-PO_2R^1$, $-PO_3R^1R^2$ wobei $R^1$ in den vorstehend aufgeführten Substituenten für H, $C_1$-$C_{18}$-Alkyl, $C_1$-$C_{18}$-Haloalkyl, $C_3$-$C_6$-Cycloalkyl, Phenyl, Benzyl oder Pyridyl steht und $R^2$ unabhängig die gleiche Bedeutung wie $R^1$ hat.

**11.** Verfahren gemäß einem oder mehreren der Ansprüche 1 bis 10, dadurch gekennzeichnet, daß R in Formel II substituiert ist mit Phenyl, Naphthyl, Pyridyl, Pyrimidyl, Pyridazinyl, Thiazolyl oder Imidazolyl, wobei diese Reste substituiert sein können mit $C_1$-$C_{12}$-Alkyl, Phenyl, Benzyl, $C_7$-$C_{14}$-Alkylphenyl, $C_8$-$C_{14}$-Alkylbenzyl, $C_1$-$C_{12}$-Alkoxy, Phenoxy, Benzyloxy, $C_7$-$C_{12}$-Alkylphenoxy, $C_8$-$C_{14}$-Alkylbenzyloxy, $C_7$-$C_{12}$-Phenoxyalkyl, $C_7$-$C_{14}$-Alkoxyphenyl, Cyclopentyl, Cyclohexyl, Cyclopropyl, $C_1$-$C_{12}$-Haloalkyl, $C_1$-$C_{12}$-Haloalkoxy, CN, OH, F, Cl, Br, J, wobei die aliphatische Reste auch unterbrochen sein können durch O, S, $NR^1$, worin $R^1$ die in Anspruch 10 angegebene Bedeutung hat, oder durch -CO oder C(O)O-.

**12.** Verfahren gemäß einem oder mehreren der Ansprüche 1 bis 11, dadurch gekennzeichnet, daß R in Formel II $C_1$-$C_6$-Alkyl, $C_3$-$C_6$-Cycloalkyl, $C_1$-$C_6$-Haloalkyl oder unsubstituiertes oder substituiertes Phenyl, Benzyl, Pyridyl, Pyrimidyl, Pyridazinyl oder Thiazolyl, wobei als Substituenten Halogen, $C_1$-$C_6$-Halogenalkyl, $C_1$-$C_6$-Halogenalkoxy, $C_1$-$C_6$-Alkyl und $C_1$-$C_6$-Alkoxy in Betracht kommen, ist.

## Claims

**1.** A process for preparing compounds of the formula I

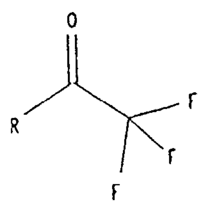

(I)

where R is an unsubstituted or substituted aliphatic or aromatic hydrocarbon radical or an unsubstituted or substituted aliphatic or aromatic heterocyclic radical, wherein an alcohol of the formula II

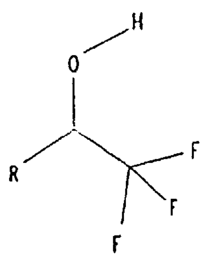

(II)

where R has the abovementioned meaning, is reacted in an aprotic solvent in the presence of 0.1 - 20 mol %, based on the alcohol of the structure II employed, of a phase-transfer catalyst, with a 1 - 20 % strength by weight

aqueous solution of compounds of the structure IV or a 1 -20 % strength by weight aqueous solution of a compound generated in situ of the structure IV

$$YO_nH \qquad (IV)$$

where Y is = Cl, Br or I and n is = an integer from 1 to 4, or of its salts, at temperatures from 0°C up to the boiling point of the mixture.

**2.** The process as claimed in claim 1, wherein a compound of the formula IV or one of its salts is employed as such in the form of the aqueous solution.

**3.** The process as claimed in claim 1 or 2, wherein an aqueous solution of a compound of the formula IV which has been prepared in situ by electrochemical means or by reaction of $H_2O_2$ with a hydrohalic acid or its salts is employed.

**4.** The process as claimed in one or more of claims 1 to 3, wherein one or more of the following conditions are fulfilled:

a) the aprotic solvent is methylene chloride, chloroform, ethyl acetate, toluene, xylene or chlorobenzene,
b) the phase-transfer catalyst is an ammonium salt of the formula III

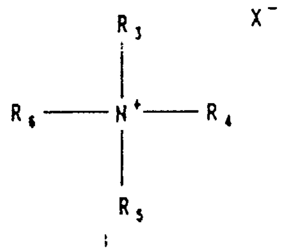
(III)

where $R^3$ - $R^6$ are the same or different and are benzyl, phenyl, $(C_1$-$C_{16})$-alkyl and $X^-$ is $OH^-$ or anions of monobasic or polybasic acids,
c) 1 - 10 mol % of phase-transfer catalyst are added, related to the alcohol II employed,
d) a 5 - 15 % strength (percent by weight) aqueous solution of a compound of the formula IV or of an alkali metal or alkaline earth metal salt of this compound is used,
e) 1 - 2 mol of oxidizing agent of the structure IV are employed per mol of alcohol of the structure II,
f) the reaction temperature is 0 to 40°C.

**5.** The process as claimed in one or more of claims 1 to 4, wherein one or more of the following conditions are fulfilled:

a) the aprotic solvent is methylene chloride, chloroform, ethyl acetate, toluene, xylene or chlorobenzene,
b) the phase-transfer catalyst is an ammonium salt of the formula III

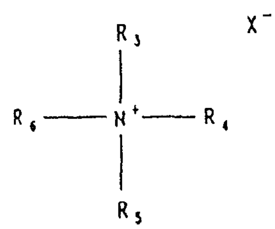
(III)

where $R^3$ - $R^6$ are the same or different and are benzyl, phenyl, $(C_1$-$C_8)$-alkyl and $X^-$ is $Cl^-$, $Br^-$ or $HSO_4^-$,
c) 1 - 10 mol % of ammonium salt of the formula III are added as the phase-transfer catalyst,
d) an aqueous solution of HOCl, $HClO_4$, HOBr, $HBr_2$, $HBrO_3$, $HIO_4$ or their salts is used as the 5 - 15 % strength

aqueous solution of a compound of the structure IV,
e) 1 - 2 mol of oxidizing agent of the structure IV are employed per mol of alcohol of the structure II,
f) the reaction temperature is 0°C to 40°C.

**6.** The process as claimed in one or more of claims 1 to 5, wherein one or more of the following conditions are fulfilled:

a) the aprotic solvent is methylene chloride, ethyl acetate, toluene, xylene or chlorobenzene,
b) the phase-transfer catalyst is benzyltrimethylammonium chloride, tetraethylammonium bromide, tetrapropylammonium bromide, tetrabutylammonium bromide, tetrabutylammonium hydrogen sulfate, hexadecyltrimethylammonium bromide or benzyltributylammonium bromide,
c) 1 - 10 mol % of ammonium salt of the formula III are added as the phase-transfer catalyst,
d) an aqueous solution of HOCl, HOBr, NaOCl, NaOBr or $Ca(OCl)_2$ is used as the 5 - 15 % strength aqueous solution of a compound of the structure IV,
e) 1 - 2 mol of oxidizing agent of the structure IV are employed per mol of alcohol of the structure II,
f) the reaction temperature is 0 to 40°C.

**7.** The process as claimed in one or more of claims 1 to 6, wherein one or more of the following conditions are fulfilled:

a) the aprotic solvent is methylene chloride, ethyl acetate, toluene, xylene or chlorobenzene,
b) the phase-transfer catalyst is tetrabutylammonium bromide or tetrabutylammonium hydrogen sulfate,
c) 1 - 10 mol % of ammonium salt of the formula III are added as the phase-transfer catalyst,
d) an aqueous solution of sodium hypobromite, sodium hypochlorite or calcium hypochlorite is used as the 5 - 15 % strength aqueous solution of a compound of the structure IV,
e) 1 - 2 mol of oxidizing agent of the structure IV are employed per mol of alcohol of the structure II,
f) the reaction temperature is 0 to 40°C.

**8.** The process as claimed in one or more of claims 1 to 7, wherein R in formula II contains, as hydrocarbon radical, 1 to 30 carbon atoms and, as heterocyclic radical, possesses 4 to 8 ring members and 1 to 4 hetero atoms.

**9.** The process as claimed in one or more of claims 1 to 8, wherein R in formula II is, as an aliphatic hydrocarbon radical, unsubstituted or substituted alkyl, alkenyl, alkynyl, cycloalkyl or cycloalkenyl, as an unsubstituted or substituted aromatic hydrocarbon radical, aryl, aralkenyl, aralkynyl or aralkyl, as an unsubstituted or substituted aliphatic heterocyclic radical, a 3- to 7-membered heterocyclic ring containing 1 to 3 hetero atoms, and, as an aromatic heterocyclic radical, a 5- or 6-membered heterocyclic ring containing 1 to 4 hetero atoms.

**10.** The process as claimed in one or more of claims 1 to 9, wherein R in formula II is substituted by $C_1$-$C_{18}$-alkyl, $C_2$-$C_{18}$-alkenyl, $C_2$-$C_{18}$-alkynyl, $C_6$-$C_{18}$-aryl, $C_7$-$C_{18}$-aralkyl, $C_7$-$C_{18}$-alkaryl, $C_8$-$C_{18}$alkarylalkyl, $C_1$-$C_{18}$-alkoxy, $C_1$-$C_{18}$-alkylthio, $C_6$-$C_{18}$-aryloxy, $C_7$-$C_{18}$-aralkoxy, $C_7$-$C_{18}$-aralkylthio, $C_7$-$C_{18}$-alkaryloxy, $C_8$-$C_{18}$-alkaralkoxy, $C_7$-$C_{18}$-aryloxyalkyl, $C_7$-$C_{18}$-alkoxyaryl, hetaryl, hetarylalkyl, alkylhetaryl, alkylhetarylalkyl, hetaryloxy, hetarylalkyloxy, hetarylalkylthio, alkylhetaryloxy, alkoxyhetaryl, alkylhetarylalkyloxy, hetaryloxyalkyl, the heterocyclic aryl radicals each having up to 8 carbon atoms and up to 4 hetero atoms, selected from the group comprising N, S and O, and the alkyl groups which may be present possessing in all up to 12 carbon atoms, $C_3$-$C_{12}$-cycloalkyl, $C_3$-$C_{12}$-cycloalkoxy, $C_3$-$C_{12}$-cycloalkenyl, $C_3$-$C_{12}$-cycloalkenyloxy, $C_1$-$C_{18}$-haloalkyl, in particular fluoroalkyl, -CN, OH, $NO_2$, F, Cl, Br, I, -NCO, $COOR^1$, $COR^1$, $-OCOR^1$, $-CONR^1R^2$, $-NR^1COR^2$, $-NR^1R^2$, $-SO_2NR^1R^2$, $-Si(R^1)_3$, $-SO_2R^1$, $-SO_3R^1$, $-PO_2R^1$ and $-PO_3R^1R^2$, where $R^1$ in the above-listed substituents is H, $C_1$-$C_{18}$-alkyl, $C_1$-$C_{18}$-haloalkyl, $C_3$-$C_6$-cycloalkyl, phenyl, benzyl or pyridyl, and $R^2$ independently has the same meaning as $R^1$.

**11.** The process as claimed in one or more of claims 1 to 10, wherein R in formula II is substituted by phenyl, naphthyl, pyridyl, pyrimidyl, pyridazinyl, thiazolyl or imidazolyl, where these radicals may be substituted by $C_1$-$C_{12}$-alkyl, phenyl, benzyl, $C_7$-$C_{14}$-alkylphenyl, $C_8$-$C_{14}$-alkylbenzyl, $C_1$-$C_{12}$-alkoxy, phenoxy, benzyloxy, $C_7$-$C_{12}$-alkylphenoxy, $C_8$-$C_{14}$-alkylbenzyloxy, $C_7$-$C_{12}$-phenoxyalkyl, $C_7$-$C_{14}$-alkoxyphenyl, cyclopentyl, cyclohexyl, cyclopropyl, $C_1$-$C_{12}$-haloalkyl, $C_1$-$C_{12}$-haloalkoxy, CN, OH, F, Cl, Br or I, where the aliphatic radicals may be interrupted by O, S, $NR^1$, where $R^1$ has the meaning given in claim 10, or by -CO- or -C(O)O-.

**12.** The process as claimed in one or more of claims 1 to 11, wherein R in formula II is $C_1$-$C_6$-alkyl, $C_3$-$C_6$-cycloalkyl, $C_1$-$C_6$-haloalkyl or unsubstituted or substituted phenyl, benzyl, pyridyl, pyrimidyl, pyridazinyl or thiazolyl, with $C_1$-$C_6$-haloalkyl, $C_1$-$C_6$-haloalkoxy, $C_1$-$C_6$-alkyl and $C_1$-$C_6$-alkoxy being suitable substituents.

## Revendications

1. Procédé de préparation de composés de formule I

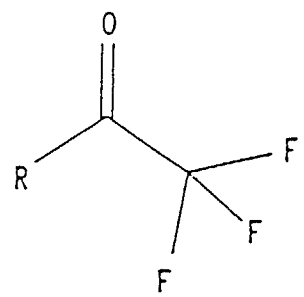

(I)

dans laquelle R est un radical hydrocarboné aliphatique ou aromatique, non substitué ou substitué, ou un radical aliphatique- ou aromatique-hétérocyclique, non substitué ou substitué,
caractérisé en ce que l'on fait réagir un alcool de formule II

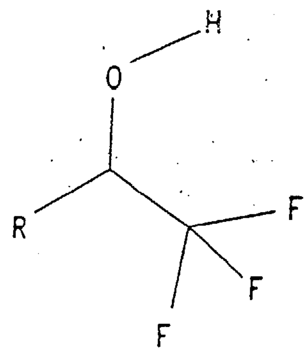

(II)

dans laquelle R a la signification donnée ci-dessus, dans un solvant aprotique, en présence de 0,1 à 20 % en moles, par rapport à l'alcool de formule II utilisé, d'un catalyseur de transfert de phases avec une solution aqueuse à 1 à 20 % d'un composé de formule IV ou une solution aqueuse de 1 à 20 % en poids d'un composé de formule IV formée *in situ*

$$YO_nH \qquad (IV)$$

dans laquelle Y = Cl, Br ou I et n est un nombre entier de 1 à 4 ou de ses sels à une température de 0 °C jusqu'au point d'ébullition du mélange.

2. Procédé selon la revendication 1, caractérisé en ce que l'on utilise un composé de formule IV ou un de ses sels tels quels sous forme de solution aqueuse.

3. Procédé selon la revendication 1 ou 2, caractérisé en ce que l'on utilise une solution d'un composé de formule IV préparé *in situ* par voie électrochimique ou par réaction de $H_2O_2$ avec un hydracide ou ses sels.

4. Procédé selon une ou plusieurs des revendications 1 ou 3, caractérisé en ce que l'on respecte une ou plusieurs des conditions suivantes :

   a) le solvant aprotique est le chlorure de méthylène, le chloroforme, l'acétate d'éthyle, le toluène, le xylène ou le chlorobenzène,
   b) le catalyseur de transfert de phases et un sel d'ammonium de formule III

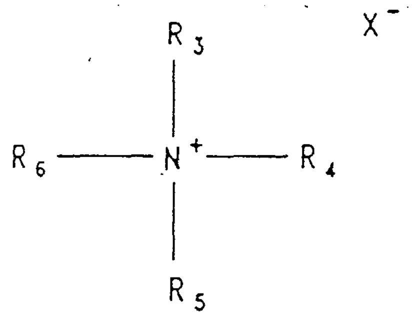

(III)

dans laquelle $R_3$-$R_6$ sont identiques ou différents et représentent benzyle, phényle, alkyle en $C_1$-$C_{16}$ et X représente OH ou des anions d'un acide mono-, respectivement polyfonctionnel,

c) on ajoute de 1 à 10 % en moles de catalyseur de transfert de phases par rapport à l'alcool II utilisé,

d) on utilise une solution aqueuse à 5 à 15 % (pourcent en poids) d'un composé de formule IV ou d'un sel alcalin ou de métaux alcalino-terreux de ce composé,

e) on utilise par mole d'alcool de formule II, 1 à 2 moles d'agent oxydant de formule IV,

f) la température de réaction est de 0 à 40 °C.

**5.** Procédé selon une ou plusieurs des revendications 1 à 4, caractérisé en ce que l'on respecte une ou plusieurs des conditions suivantes :

a) le solvant aprotique est le chlorure de méthylène, le chloroforme, l'acétate d'éthyle, le toluène, le xylène ou le chlorobenzène,

b) le catalyseur de transfert de phases est un sel d'ammonium de formule III,

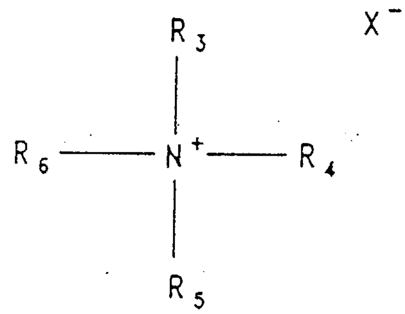

(III)

dans laquelle $R_3$-$R_6$ sont identiques ou différents et représentent benzyle, phényle, alkyle en $C_1$-$C_8$, $X^-$ représente $Cl^-$, $Br^-$ ou $HSO_4^-$,

c) on ajoute 1 à 10 % en moles de sel d'ammonium de formule III comme catalyseur de transfert de phases,

d) on utilise comme solution aqueuse à 5 à 15 % d'un composé de formule IV une solution aqueuse de HOCl, $HClO_4$, HOBr, $HBrO_2$, $HBrO_3$, $HIO_4$, ou de leurs sels,

e) on utilise par mole d'alcool de formule II, 1 à 2 moles d'agent oxydant de formule IV,

f) la température de réaction est de 0 à 40 °C.

**6.** Procédé selon une ou plusieurs des revendications 1 à 5, caractérisé en ce que l'on respecte une ou plusieurs des conditions suivantes :

a) le solvant aprotique est le chlorure de méthylène, l'acétate d'éthyle, le toluène, le xylène ou le chlorobenzène,

b) le catalyseur de transfert de phases est le chlorure de. benzyltriméthylammonium, le bromure de tétraéthylammonium, le bromure de tétrapropylammonium, le bromure de tétrabutylammonium, le bisulfate de tétrabutylammonium, le bromure d'hexadécyltriméthylammonium ou le bromure de benzyltributylammonium,

c) on ajoute de 1 à 10 % en moles de sel d'ammonium de formule III comme catalyseur de transfert de phases,

d) on utilise comme solution aqueuse à 5-15 % d'un composé de formule IV, une solution aqueuse de HOCl,

$HClO_4$, HOBr, NaOCl, NaOBr ou $Ca(OCl)_2$,
e) on utilise par mole d'alcool de formule II, 1 à 2 moles d'agent oxydant de formule IV,
f) la température de réaction est de 0 à 40 °C.

**7.** Procédé selon une ou plusieurs des revendications 1 à 6, caractérisé en ce que l'on respecte une ou plusieurs des conditions suivantes :

a) le solvant aprotique est le chlorure de méthylène, l'acétate d'éthyle, le toluène, le xylène ou le chlorobenzène,
b) le catalyseur de transfert de phases est le bromure de tétrabutylammonium ou le bisulfate de tétrabutylammonium,
c) on ajoute de 1 à 10 % en moles de sel d'ammonium de formule III comme catalyseur de transfert de phases,
d) on utilise comme solution aqueuse à 5-15 % d'un composé de formule IV, une solution aqueuse d'hypobromite de sodium, d'hypochlorite de sodium ou d'hypochlorite de calcium,
e) on utilise par mole d'alcool de formule II 1 à 2 moles d'agent oxydant de formule IV,
f) la température de réaction est de 0 à 40 °C.

**8.** Procédé selon une ou plusieurs des revendications 1 à 7, caractérisé en ce que R dans la formule II en tant que radical hydrocarboné contient de 1 à 30 atomes de carbone et en tant que radical hétérocyclique présente 4 à 8 chaînons de cycles et 1 à 4 hétéroatomes.

**9.** Procédé selon une ou plusieurs des revendications 1 à 8, caractérisé en ce que R dans la formule II en tant que radical hydrocarboné aliphatique non substitué ou substitué représente alkyle, alcényle, alcynyle, cycloalkyle ou cycloalcényle, en tant que radical hydrocarboné aromatique non substitué ou substitué représente aryle, aralcényle, aralcynyle ou aralkyle, en tant que radical aliphatique-hétérocyclique non substitué ou substitué représente un cycle hétérocyclique à 3 à 7 chaînons contenant 1 à 3 hétéroatomes et en tant que radical hétérocyclique-aromatique, un cycle hétérocyclique à 5 ou 6 chaînons contenant 1 à 4 hétéroatomes.

**10.** Procédé selon une ou plusieurs des revendications 1 à 9, caractérisé en ce que R dans la formule II est substitué par alkyle en $C_1$-$C_{18}$, alcényle en $C_2$-$C_{18}$, alcynyle en C2-C18, aryle en C6-C18, aralkyle en $C_7$-$C_{18}$, alcaryle en $C_7$-$C_{18}$, alcarylalkyle en $C_7$-$C_{18}$, alcoxy en $C_1$-$C_{18}$, alkylthio en $C_1$-$C_{18}$, aryloxy en $C_6$-$C_{18}$, aralcoxy en $C_7$-$C_{18}$, aralkylthio en $C_7$-$C_{18}$, alcaryloxy en $C_7$-$C_{18}$, alcaralcoxy en $C_8$-$C_{18}$, aryloxyalkyle en $C_7$-$C_{18}$, alcoxyaryle en $C_7$-$C_{18}$, hétaryle, hétarylalkyle, alkylhétaryle, alkylhétarylalkyle, hétaryloxy, hétarylalkyloxy, hétarylalkylthio, alkylhétaryloxy, alcoxyhétaryle, alkylhétarylalkyloxy, hétaryloxyalkyle, les radicaux aryle hétérocycliques ayant chacun jusqu'à 8 atomes de carbone et présentent chacun jusqu'à 4 hétéroatomes pris dans le groupe constitué de N, S et O, et les groupes alkyle éventuellement présents ont au total jusqu'à 12 atomes de carbone, cycloalkyle en $C_3$-$C_{12}$, cycloalcoxy en $C_3$-$C_{12}$, cycloalcényle en $C_3$-$C_{12}$, cycloalcényloxy en $C_3$-$C_{12}$, haloalkyle en $C_1$-$C_{18}$, plus particulièrement fluoralkyle, -CN, OH, $NO_2$, F, Cl, Br, I, -NCO, $COOR^1$, $COR^1$, $-OCOR^1$, $-CONR^1R^2$, $-NR^1COR^2$, $-NR^1R^2$,$-SO_2NR^1R^2$, $-Si(R^1)_3$, $-SO_2R^1$, $-SO_3R^1$, $-PO_2R^1$, $-PO_3R^1R^2$. $R^1$, dans les substituants précités, représente H, alkyle en $C_1$-$C_{18}$, haloalkyle en $C_1$-$C_{18}$, cycloalkyle en $C_3$-$C_6$, phényle, benzyle ou pyridyle et $R^2$, indépendamment, a la même signification que $R^1$.

**11.** Procédé selon une ou plusieurs des revendications 1 à 10, caractérisé en ce que R dans la formule II est substitué par phényle, naphtyle, pyridyle, pyrimidyle, pyridazinyle, thiazolyle ou imidazolyle, ces radicaux pouvant être substitués par alkyle en $C_1$-$C_{12}$, phényle, benzyle, (alkyl en $C_7$-$C_{14}$)phényle, (alkyl en $C_8$-$C_{14}$)benzyle, alcoxy en $C_1$-$C_{12}$, phénoxy, benzyloxy, (alkyle en $C_7$-$C_{12}$)phénoxy, (alkyl en $C_8$-$C_{14}$)benzyloxy, phénoxyalkyle en $C_7$-$C_{12}$, (alcoxy en $C_7$-$C_{14}$)phényle, cyclopentyle, cyclohexyle, cyclopropyle, haloalkyle en $C_1$-$C_{12}$, haloalcoxy en $C_1$-$C_{12}$, CN, OH, F, Cl, Br, I, les radicaux aliphatiques pouvant aussi être interrompus par O, S, $NR^1$, $R^1$ ayant la signification donnée dans la revendication 10, ou aussi par -CO ou C(O)O-.

**12.** Procédé selon une ou plusieurs des revendications 1 à 11, caractérisé en ce que R dans la formule II représente alkyle en $C_1$-$C_6$, cycloalkyle en $C_3$-$C_6$, haloalkyle en $C_1$-$C_6$, ou phényle, benzyle, pyridyle, pyrimidyle, pyridazinyle ou thiazolyle non substitué ou substitué, en envisageant comme substituants des halogènes, haloalkyle en $C_1$-$C_6$, haloalcoxy en $C_1$-$C_6$, alkyle en $C_1$-$C_6$ et alcoxy en $C_1$-$C_6$.